# EUROPÄISCHE PATENTSCHRIFT

(11) **EP 0 360 041 B1**
(45) Veröffentlichungstag und Bekanntmachung des Hinweises auf die Patenterteilung: **28.04.1993**
(21) Anmeldenummer: 89115908.9
(22) Anmeldetag: 29.08.1989
(51) Int. Cl.: B01D 3/40

(54) **Verfahren und Vorrichtung zur Optimierung des Betriebes einer mit einem Seitenkocher versehenen Abtriebskolonne**
Method and apparatus for optimizing the operation of a rectification column provided with a lateral boiler
Procédé et dispositif pour optimiser le fonctionnement d'une colonne de rectification contenant un reboiler latéral

(30) Priorität: 23.09.1988 DE 3832340
(43) Veröffentlichungstag der Anmeldung: 28.03.1990
(73) Patentinhaber: Krupp Koppers GmbH, 45143 Essen (DE)
(72) Erfinder: Emmrich, Gerd, D-4300 Essen 1 (DE); Schneider, Hans-Christoph, D-4320 Hattingen (DE); Rüdel, Ulrich, D-4200 Oberhausen 1 (DE)

(56) Entgegenhaltungen:
- DE-A- 2 648 638
- FR-A- 2 578 438
- US-A- 2 981 662
- US-A- 4 559 109

## Beschreibung

Die Erfindung betrifft ein Verfahren und eine Vorrichtung zur Aufarbeitung eines Extraktes, der bei der Extraktion kohlenwasserstoffhaltiger Einsatzprodukte mit N-substituierten Morpholinen, deren Substituenten nicht mehr als sieben C-Atome aufweisen, als selektivem Lösungsmittel anfällt.

Die Extraktion kohlenwasserstoffhaltiger Einsatzprodukte mit den vorstehend genannten N-substituierten Morpholinen, insbesondere mit N-Formylmorpholin, als selektivem Lösungsmittel ist bereits seit längerer Zeit bekannt und wird heute im großtechnischen Maßstab vor allem zur Gewinnung hochreiner Aromaten eingesetzt. Das Verfahren ist aber auch zur Gewinnung anderer Stoffklassen, wie z.B. der Olefine und Diolefine, geeignet, wobei sowohl die Extraktivdestillation als auch die Flüssig-Flüssig-Extraktion zur Anwendung gelangen können. Die aus den entsprechenden Einsatzprodukten zu gewinnenden Kohlenwasserstoffe reichern sich dabei zusammen mit der Hauptmenge des Lösungsmittels im Extrakt an und müssen in einer der Extraktionsstufe nachgeschalteten Abtriebskolonne vom Lösungsmittel abgetrennt werden. Hierbei werden die zu gewinnenden Kohlenwasserstoffe über Kopf aus der Abtriebskolonne abdestilliert, während das Lösungsmittel als Sumpfprodukt dieser Kolonne anfällt und von dort seiner Wiederverwendung zugeführt werden kann. Hierbei kann die Abtriebskolonne zu ihrer Beheizung neben den üblichen Sumpfumlaufkochern noch einen Seitenkocher aufweisen, der in erster Linie den auf die Abtriebskolonne aufgegebenen Rückfluß wieder verdampfen soll.

Beim Betrieb derartiger Abtriebskolonnen wurde nun insbesondere in Verbindung mit der Verwendung von N-Formylmorpholin als selektivem Lösungsmittel beobachtet, daß sich das Lösungsmittel auf dem Boden der Abtriebskolonne aufkonzentriert, der oberhalb des Kocheraustrittes liegt und gleichzeitig Abzugsboden für den Seitenkocher ist. Dadurch wird auf diesem Boden die Siedetemperatur erhöht und somit die Wärmeleistung des Bodens laufend verschlechtert. Durch eine Erhöhung des Rückflusses kann die Wärmeabgabe des Seitenkochers zwar vergrößert werden. Dies bringt jedoch keinen Vorteil, da die Sumpfumlaufkocher der Abtriebskolonne weiterhin die gleiche Wärmemenge zuführen müssen und somit deren Gesamtwärmebilanz nicht verbessert wird.

Der Erfindung liegt deshalb die Aufgabe zugrunde, bei einem Verfahren der eingangs genannten Art den Betrieb der Abtriebskolonne so zu optimieren, daß die vorstehend geschilderten Nachteile vermieden werden und die Funktion des Seitenkochers wesentlich verbessert werden kann.

Diese Aufgabe wird durch Anwendung einer mit einem Seitenkocher versehenen Abtriebskolonne gelöst, bei der der Flüssigkeitsabzug zum Seitenkocher über einen oberhalb des Aufgabebodens angeordneten Kaminboden erfolgt, wobei zwischen 5 und 30 Vol-% der dem Kaminboden zulaufenden Flüssigkeit nicht in den Seitenkocher abgezogen, sondern direkt auf den darunter liegenden Boden abgeleitet werden und wobei das über Kopf aus dem Seitenkocher austretende Dampf-Flüssigkeitsgemisch in der Weise in die Abtriebskolonne zurückgeführt wird, daß es entweder auf den Aufgabeboden oder auf den unterhalb des Kaminbodens befindlichen Boden aufgegeben wird.

Das erfindungsgemäße Verfahren sieht also eine Aufteilung der vom Kaminboden abzuziehenden Flüssigkeit vor, wobei zwischen 5 und 30 Vol.-% dieser Flüssigkeit direkt auf den darunter liegenden Boden abgeleitet werden und der Rest dem Seitenkocher zugeführt wird. Zu diesem Zweck kann der Kaminboden mit einem Überlaufwehr versehen sein, hinter dem eine Ablauftasse für den Flüssigkeitsabzug zum Seitenkocher und ein Ablaufschacht für den Flüssigkeitsabzug auf den darunter liegenden Boden angeordnet sind. Die prozentuale Aufteilung der gesamten Wehrlänge in diese beiden Teilbereiche entspricht dabei jeweils der gewünschten prozentualen Aufteilung der Flüssigkeitsmenge. Durch diese Maßnahme gelingt es auf einfache Art und Weise sicherzustellen, daß auch bei unterschiedlichen Gesamtflüssigkeitsmengen auf dem Kaminboden jeweils die gewünschte prozentuale Aufteilung der beiden Flüssigkeitsteilströme eingehalten wird.

Durch den Abzug einer Teilmenge der Flüssigkeit vom Kaminboden auf den darunter liegenden Boden gelingt es, auf diesem Boden einen Wascheffekt hervorzurufen, der vorzugsweise das Lösungsmittel vom Kaminboden fernhält und in den Unterteil der Abtriebskolonne zurückführt. Durch diese Verringerung der Lösungsmittelkonzentration auf dem Kaminboden wird die Siedetemperatur der in den Seitenkocher einzuleitenden Flüssigkeit herabgesetzt und die Kocherleistung gesteigert. Das heißt, ein höherer Antei des auf die Abtriebskolonne aufgegebenen Rückflusses kann im Seitenkocher verdampft werden.

Für die Rückführung des über Kopf aus dem Seitenkocher austretenden Dampf-Flüssigkeitsgemisches in die Abtriebskolonne sieht das erfindungsgemäße Verfahren zwei Varianten vor.

Bei der ersten Variante wird dieses Gemisch auf den Aufgabeboden der Abtriebskolonne wieder aufgegeben. Als Aufgabeboden wird hierbei jener Boden bezeichnet, auf den der aufzuarbeitende Extrakt in die Abtriebskolonne eingeleitet wird. Die Anwendung dieser Verfahrensvariante ist zweckmäßig, wenn der Abtriebskolonne viel Wärme zugeführt werden soll. Hierbei ist es allerdings notwendig, daß zwischen dem Aufgabeboden und dem Kaminboden mindestens drei normale Böden angeordnet sind.

Bei der zweiten Variante wird das Dampf-Flüssigkeitsgemisch aus dem Seitenkocher auf den unterhalb des Kaminbodens befindlichen Boden aufgegeben.

In beiden Fällen ist es zweckmäßig, wenn die Wiedereinleitung des Dampf-Flüssigkeitsgemisches in die Abtriebskolonne tangential erfolgt, da hierdurch die Trennung von Dampf- und Flüssigkeitsbestandteilen verbessert wird.

Weitere Einzelheiten des erfindungsgemäßen Verfahrens sowie der zu seiner Durchführung geeigneten Vorrichtung sollen nachfolgend an Hand der Abbildungen erläutert werden. Hierbei zeigen in vereinfachter schematischer Darstellung:
- Fig. 1: einen Schnitt durch eine für die Durchführung des erfindungsgemäßen Verfahrens geeignete Abtriebskolonne und
- Fig. 2: eine Draufsicht auf den Kaminboden dieser Kolonne.

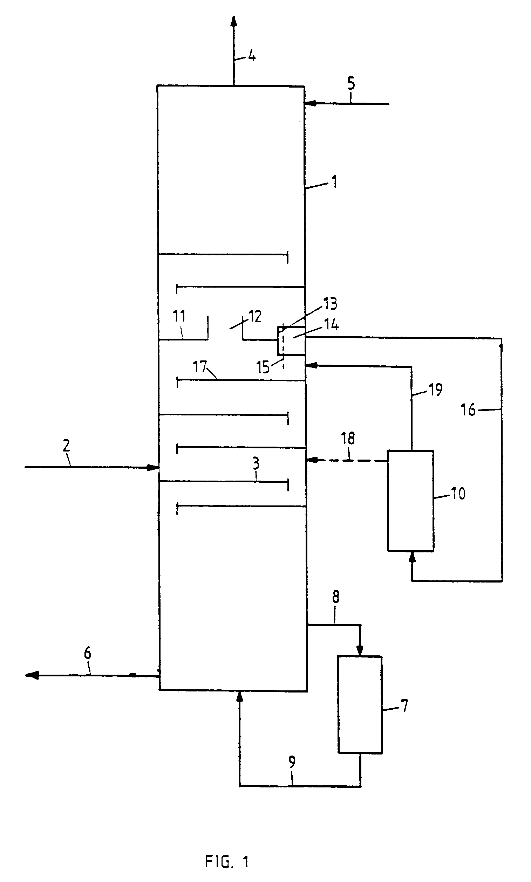

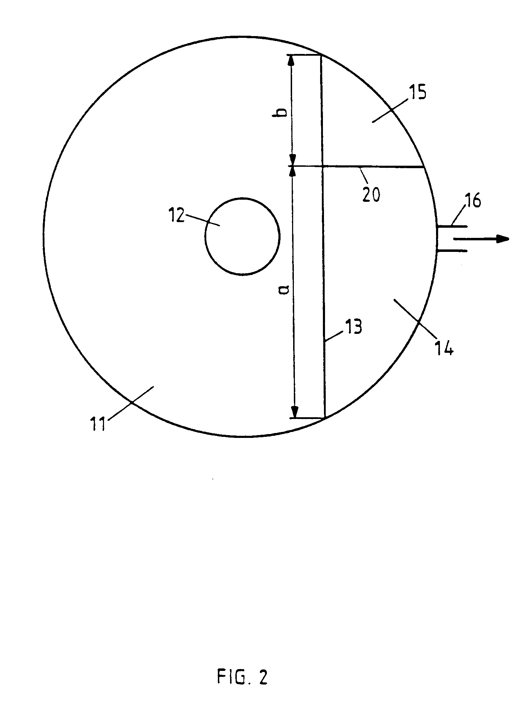

Bei der in Fig. 1 in vereinfachter schematischer Darstellung wiedergegebenen Abtriebskolonne 1 wird der von der vorgeschalteten Extraktionsstufe kommende Extrakt über die Leitung 2 im mittleren Bereich in diese Kolonne eingeleitet. Der Boden, auf den dabei die Einleitung des Extraktes erfolgt, wird als Aufgabeboden 3 bezeichnet. In der Abtriebskolonne 1 erfolgt wie üblich die destillative Auftrennung des Extraktes, wobei die zu gewinnenden Kohlenwasserstoffe über die Leitung 4 als Kopfprodukt aus der Abtriebskolonne 1 abgezogen werden. Eine Teilmenge der abgezogenen Kohlenwasserstoffe wird über die Leitung 5 als Rückfluß wieder auf den Oberteil der Abtriebskolonne 1 aufgegeben. Der Abzug des von den zu gewinnenden Kohlenwasserstoffen befreiten Lösungsmittels aus dem Sumpf der Abtriebskolonne 1 erfolgt über die Leitung 6, durch welche das Lösungsmittel seiner Wiederverwendung in der Abbildung nicht dargestellten Extraktionsstufe zugeführt werden kann. Die Beheizung der Abtriebskolonne 1 kann in an sich bekannter Weise durch einen oder mehrere Sumpfumlaufkocher 7 erfolgen, die durch den Flüssigkeitskreislauf über die Leitungen 8 und 9 mit dem Sumpf der Abtriebskolonne 1 verbunden sind. Die erfindungsgemäße Abtriebskolonne 1 ist außerdem mit dem Seitenkocher 10 ausgerüstet, der in erster Linie der Wiederverdampfung des über die Leitung 5 auf die Abtriebskolonne 1 aufgegebenen Rückflusses dienen soll. Selbstverständlich ist dabei auch der Seitenkocher 10 mit den für seine Beheizung notwendigen Einrichtungen versehen, die in der Abbildung jedoch nicht dargestellt sind. Der Flüssigkeitsabzug zum Seitenkocher 10 erfolgt vom Kaminboden 11, der oberhalb des Aufgabebodens 3 angeordnet ist. Der Kaminboden 11 weist, wie sein Name bereits sagt, einen Kamin 12 sowie erfindungsgemäß das Überlaufwehr 13 auf. Die auf den Kaminboden 11 von oben herabfließende und sich dort ansammelnde Flüssigkeit fließt, sobald der entsprechende Flüssigkeitsstand erreicht ist, über das Überlaufwehr 13 ab und gelangt in die dahinter liegende Ablauftasse 14 bzw. in den Ablaufschacht 15. Auf weitere Einzelheiten dieses Flüssigkeitsabzuges wird weiter unten noch im Zusammenhang mit Fig. 2 eingegangen werden. Der in die Ablauftasse 14 abgeflossene Teilstrom der Flüssigkeit wird über die Leitung 16 in den Unterteil des Seitenkochers 10 eingeleitet, während der restliche Teilstrom der Flüssigkeit über den Ablaufschacht 15 auf den unterhalb des Kaminbodens 11 befindlichen Boden 17 abgeleitet wird.

Das über Kopf aus dem Seitenkocher 10 entweichende Dampf-Flüssigkeitsgemisch kann entweder über die Leitung 18 auf den Aufgabeboden 3 oder über die Leitung 19 auf den unmittelbar unter dem Kaminboden 11 befindlichen Boden 17 in die Abtriebskolonne 1 wieder eingeleitet werden. Daß es sich hierbei um zwei Alternativen handelt, die nicht gleichzeitig angewandt werden können, ist in der Abbildung dadurch kenntlich gemacht worden, daß die Leitung 18 unterbrochen gezeichnet wurde. Sofern die Wiedereinleitung über die Leitung 18 erfolgt, sollen zwischen dem Aufgabeboden 3 und dem Kaminboden 11 mindestens drei Böden angeordnet sein, was in der Abbildung angedeutet wurde.

Fig. 2 zeigt eine Draufsicht auf den Kaminboden 11 mit dem dazugehörigen Kamin 12 und dem Überlaufwehr 13. Hinter dem Überlaufwehr 13 befinden sich die Ablauftasse 14 und der Ablaufschacht 15, die durch die Trennwand 20 voneinander getrennt sind. Die Gesamtlänge des Überlaufwehres 13 ist dabei so in die Abschnitte a und b aufgeteilt, wie dies der jeweils gewünschten Aufteilung der beiden Flüssigkeitsteilströme entspricht. Das heißt, der Abschnitt b, hinter dem der zum darunter liegenden Boden 17 führende Ablaufschacht 15 liegt, weist eine Länge von 5 - 30% der Gesamtlänge des Überlaufwehres 13 auf. Dies hat zur Folge, daß über diesen Abschnitt zwischen 5 und 30 Vol.-% der Flüssigkeit vom Kaminboden 11 in den Ablaufschacht 15 fließen, während die übrige Flüssigkeit über den Abschnitt a in die Ablauftasse 14 gelangt und von dort über die Leitung 16 zum Seitenkocher 10 abgezogen wird.

Da es sich bei den in Fig. 1 und 2 dargestellten Abbildungen nur um schematische Darstellungen handelt, entsprechen die dort wiedergegebenen Größenverhältnisse nicht den in der Praxis vorhandenen Größenverhältnissen. Selbstverständlich kann die Abtriebskolonne 1 im Gegensatz zur Darstellung in Fig. 1 auch in ihrem Ober- und Unterteil weitere Böden aufweisen. Hierbei kann es sich um Abscheideböden üblicher Bauart handeln. Außerdem ist diese Kolonne in der Praxis natürlich mit den notwendigen Meß- und Regeleinrichtungen zur Steuerung ihres Betriebes versehen.

## Patentansprüche

1. Verfahren zur Aufarbeitung eines Extraktes, der bei der Extraktion kohlenwasserstoffhaltiger Einsatzprodukte mit N-substituierten Morpholinen, deren Substituenten nicht mehr als sieben C-Atome aufweisen, als selektivem Lösungsmittel anfällt, in einer mit einem Seitenkocher versehenen Abtriebskolonne, bei der der Flüssigkeitsabzug zum Seitenkocher über einen oberhalb des Aufgabebodens angeordneten Kaminboden erfolgt, wobei zwischen 5 und 30 Vol.-% der dem Kaminboden zulaufenden Flüssigkeit nicht in den Seitenkocher abgezogen, sondern direkt auf den darunter liegenden Boden abgeleitet werden und wobei das über Kopf aus dem Seitenkocher austretende Dampf-Flüssigkeitsgemisch in der Weise in die Abtriebskolonne zurückgeführt wird, daß es entweder auf den Aufgabeboden oder auf den unterhalb des Kaminbodens befindlichen Boden aufgegeben wird.

2. Verfahren nach Anspruch 1, dadurch gekannzeichnet, daß der Wiedereintritt des Dampf-Flüssigkeitsgemisches aus dem Seitenkocher in die Abtriebskolonne tangential erfolgt.

3. Verfahren nach den Ansprüchen 1 und 2, dadurch gekennzeichnet, daß bei einer Rückführung des Dampf-Flüssigkeitsgemisches aus dem Seitenkocher auf den Aufgabeboden der Abtriebskolonne zwischen diesem und dem Kaminboden mindestens drei Böden vorhanden sein müssen.

4. Vorrichtung zur Durchführung des Verfahrens nach den Ansprüchen 1 bis 3, dadurch gekennzeichnet, daß in der Abtriebskolonne (1) oberhalb des Aufgabebodens (3) ein Kaminboden (11) angeordnet ist, der mit einem in zwei Abschnitte aufgeteilten Überlaufwehr (13) versehen ist, wobei sich hinter dem einen Abschnitt eine Ablauftasse (14) für den Abzug zum Seitenkocher (10) und hinter dem anderen Abschnitt ein zum darunter liegenden Boden (17) führender Ablaufschacht (15) befinden und wobei die Aufteilung in die beiden Abschnitte der gewünschten Aufteilung der beiden vom Kaminboden (11) abzuziehenden Flüssigkeitsströme entspricht.

## Claims

1. Method for work-up of an extract, which is produced in the extraction of hydrocarbon-containing feedstock with N-substituted morpholines, the substituents of which have no more than seven C atoms, as selective solvent, in a stripping column furnished with a side reboiler, in which the liquid withdrawal to the side reboiler is carried out via a chimney tray arranged above the feed tray, between 5 and 30% by volume of the liquid flowing to the chimney tray not being withdrawn into the side reboiler but being passed directly to the tray below and the vapour/liquid mixture leaving via the head from the side reboiler being returned to the stripping column in such a manner that it is either applied to the feed tray or to the tray located beneath the chimney tray.

2. Method according to Claim 1, characterised in that the re-entry of the vapour/liquid mixture from the side reboiler into the stripping column is carried out tangentially.

3. Method according to Claims 1 and 2, characterised in that when the vapour/liquid mixture from the side reboiler is returned to the feed tray of the stripping column, at least three trays must be present between the feed tray and the chimney tray.

4. Apparatus for carrying out the method according to Claims 1 to 3, characterised in that in the stripping column (1), a chimney tray (11) is arranged above the feed tray (3), which chimney tray is furnished with an overflow weir (13) divided into two sections, an outflow pond (14) being located downstream of the one section for the withdrawal to the side reboiler (10) and an outflow shaft (15) leading to the tray (17) situated below the other section being located downstream thereof, the division into the two sections corresponding to the desired division of the two liquid streams to be withdrawn from the chimney tray (11).

## Revendications

1. Procédé pour la préparation d'un extrait qu'on obtient dans le traitement d'extraction de produits de départ contenant des hydrocarbures en utilisant comme solvant sélectif des morpholines N-substituées dont les substituants ne contiennent pas plus de sept atomes de C, dans lequel le soutirage du liquide envoyé au bouilleur latéral s'effectue par l'intermédiaire d'un plateau à cheminée disposé au-dessus du plateau d'alimentation, une proportion d'entre 5 et 30 % en volume du liquide qui arrive sur le plateau à cheminée étant, non pas soutirée et envoyée au bouilleur latéral mais directement déviée vers le plateau situé au-dessous, et le mélange vapeur-liquide qui sort en tête du bouilleur latéral étant renvoyé à la colonne de rectification de manière à être introduit, soit sur le plateau d'alimentation soit sur le plateau situé au-dessous du plateau à cheminée.

2. Procédé selon la revendication 1,
caractérisé en ce que la rentrée du mélange vapeur-liquide du bouilleur latéral dans la colonne de rectification s'effectue tangentiellement.

3. Procédé selon les revendications 1 et 2,
caractérisé en ce que, dans le cas où le renvoi du mélange vapeur-liquide provenant du houilleur latéral s'effectue sur le plateau d'alimentation de la colonne de rectification, il faut prévoir au moins trois plateaux entre le plateau d'alimentation et le plateau à cheminée.

4. Dispositif pour la mise en oeuvre du procédé selon les revendications 1 à 3,
caractérisé en ce que, dans la colonne de rectification (1), est agencé, au-dessus du plateau d'alimentation (3), un plateau à cheminée (11) qui est muni d'un déversoir de trop-plein (13) partagé en deux segments, et il est prévu, en aval de l'un des segments, une rigole d'écoulement (14) pour le soutirage envoyé au bouilleur latéral (10) et, en aval de l'autre segment, un puits d'écoulement (15) qui mène au plateau (17) situé au-dessous, le partage en les deux segments correspondant au partage désiré des courants de liquide qui doivent être soutirés du plateau à cheminée (11).
